# FASCICULE DE BREVET EUROPEEN

(11) **EP 0 966 987 B1**
(45) Date de publication et mention de la délivrance du brevet: **01.09.2004**
(21) Numéro de dépôt: 99401560.0
(22) Date de dépôt: 23.06.1999
(51) Int. Cl.: A61N 1/365

(54) **Stimulateur cardiaque/défibrillateur implantable asservi, de type multisite**
Multi-Ort, parameter- gesteuerter, implantierbarer Herzschrittmacher / Defribrillator
Multi-site, parameter controlled, implantable pacemaker / defibrillator

(30) Priorité: 26.06.1998 FR 9808118
(43) Date de publication de la demande: 29.12.1999
(73) Titulaire: ELA MEDICAL (Société anonyme), 92541 Montrouge (FR)
(72) Inventeur: Bonnet, Jean-Luc, 92120 Montrouge (FR); Limousin, Marcel, 75014 Paris (FR)
(74) Mandataire: Dupuis-Latour, Dominique

(56) Documents cités:
- EP-A- 0 654 285
- EP-A- 0 804 939
- US-A- 5 003 975
- US-A- 5 154 170
- US-A- 5 330 505
- US-A- 5 423 869
- US-A- 5 476 485
- US-A- 5 562 711

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Elle concerne plus précisément les appareils dont le fonctionnement est asservi à un paramètre recueilli par un capteur. Ces appareils sont connus pour adapter leurs actions, par exemple la fréquence de stimulation, à la valeur mesurée ou calculée d'un paramètre représentatif des besoins métaboliques du porteur de l'appareil.

Le capteur utilisé est le plus souvent un capteur de ventilation-minute (souvent dénommé capteur MV ou capteur VE).

Le EP-A-0 804 939 décrit un stimulateur dans lequel les signaux délivrés par un capteur de ventilation-minute sont utilisés, outre l'asservissement, à des fins de diagnostic de la décompensation de l'insuffisance cardiaque et agir de façon adaptée sur la programmation du stimulateur.

Mais ce dispositif présente l'inconvénient de ne pas prendre en compte le niveau réel d'activité du patient. En effet, il est connu que les insuffisants cardiaques ont une fonction ventilatoire altérée du fait de leur pathologie. De ce fait, ces patients vont présenter à l'effort des niveaux de consommation d'oxygène (VO₂) moindres que pour des patients sains ; en revanche, leur fréquence cardiaque et leur ventilation-minute augmenteront significativement plus que pour les patients sains, pour un même niveau d'activité.

Ainsi, une variation dans le temps, relative ou absolue, des signaux détectés par le capteur de ventilation-minute (ou, de façon plus générale, par le capteur physiologique censé donner une représentation adéquate des besoins métaboliques du patient), peut être due à l'insuffisance cardiaque, mais aussi à un simple changement du niveau d'activité du patient.

Il a été proposé, par exemple dans le EP-A-0 750 920 (Ela Médical) de combiner les informations délivrées par deux capteurs, l'un physiologique (typiquement un capteur de ventilation-minute), l'autre d'activité (typiquement un accéléromètre, souvent dénommé capteur G). Les deux capteurs opèrent une "surveillance croisée" de leurs indications respectives, et le stimulateur combine les informations des deux capteurs pour asservir la fréquence cardiaque. Toutefois, ce dispositif ne prend pas en compte l'évolution sur le long terme de la pathologie du patient, qui peut présenter au cours du temps des périodes de plus ou moins grande décompensation cardiaque. La pathologie peut en effet évoluer en fonction de différents facteurs tels que l'efficacité de la stimulation cardiaque, l'efficacité des médicaments, ou l'alimentation, ou encore les arythmies auriculaires et/ou ventriculaires.

Il en est de même pour le US-A-5 562 711, qui décrit un dispositif combinant les informations délivrées par les capteurs MV et G pour affiner la relation entre demande métabolique et fréquence de stimulation en prenant les avantages respectifs des deux capteurs et en les combinant. Mais la question de l'évaluation de la décompensation cardiaque n'est jamais abordée par ce document.

L'un des buts de la présente invention est de proposer un stimulateur qui permette de suivre l'évolution du patient au cours du temps de manière à donner une représentation adéquate des besoins métaboliques réels de celui-ci. En cas d'aggravation ou d'amélioration de la situation du patient, le stimulateur pourra avantageusement modifier son fonctionnement, par exemple par reprogrammation de certaines de ses fonctions.

Le dispositif de l'invention est du type à double capteur décrit dans le US-A-5 562 711 précité, c'est-à-dire comportant au moins un capteur d'effort, mesurant un paramètre à prépondérance physiologique et délivrant un signal fonction de l'effort développé par un patient porteur du dispositif, au moins un capteur d'activité, mesurant un paramètre à prépondérance physique, et des moyens d'analyse de tendance, aptes à : a) mesurer périodiquement par les deux capteurs des couples de valeurs correspondant à un niveau d'effort donné développé par le patient ; et b) établir une caractéristique fonction des couples de valeurs ainsi mesurés.

De façon caractéristique de l'invention les moyens d'analyse de tendance sont également aptes à: c) évaluer le degré de décompensation cardiaque du porteur de l'appareil d'après l'évolution au cours du temps de cette caractéristique, en recherchant un éventuel accroissement des signaux délivrés par le capteur d'effort par rapport aux signaux délivrés par le capteur d'activité.

Très avantageusement, les moyens d'analyse sont également aptes à : d) modifier la programmation du dispositif lorsque les résultats de l'étape c) indiquent un accroissement au-delà d'un seuil donné des signaux délivrés par le capteur d'effort par rapport aux signaux délivrés par le capteur d'activité.

L'invention est notamment applicable à un stimulateur de type "multisite", c'est-à-dire dans lequel des électrodes sont placées en une pluralité de sites myocardiques distincts de stimulation, la commande de la stimulation et/ou la configuration des sites sont modifiées lorsque les résultats de l'étape c) indiquent un accroissement au-delà d'un seuil donné des signaux délivrés par le capteur d'effort par rapport aux signaux délivrés par le capteur d'activité.

Les moyens d'analyse peuvent à l'étape c) évaluer le décalage de la caractéristique dans le sens des efforts croissants, ou encore évaluer l'accroissement de la pente effort/activité de la caractéristique. Cette évaluation peut notamment être opérée régulièrement (typiquement, quotidiennement).

On va maintenant décrire un exemple de mise en oeuvre de l'invention, en référence à la figure unique annexée qui représente la caractéristique ventilation-minute/activité, déterminée pour deux périodes consécutives différentes.

On va décrire l'invention dans le cadre d'un stimulateur de type multisite configurable, par exemple celui décrit dans le EP-A-0 925 806 (publié le 30.06.99), au nom d'Ela Médical. Les enseignements de cette demande sont notamment mis en oeuvre dans l'appareil *Chorum 7336* commercialisé par Ela Médical.

Typiquement, une sonde endocavitaire est placée dans le ventricule droit (technique classique en stimulation cardiaque) et une seconde sonde est placée au niveau du ventricule gauche (par exemple par l'intermédiaire du sinus coronaire et du réseau veineux coronaire). Des sondes supplémentaires peuvent être également mises en place pour permettre une stimulation atriale, simple ou double, en supplément de la double stimulation ventriculaire.

Le but de l'invention est d'évaluer à intervalles périodiques le degré de décompensation du patient sur la base de l'altération de la fonction ventilatoire associée à la pathologie cardiaque par rapport aux signaux délivrés par le capteur d'activité.

L'invention propose essentiellement de combiner l'information de deux capteurs, l'un physiologique et l'autre d'activité, pour déterminer si une variation d'amplitude du capteur physiologique au cours du temps résulte d'un simple changement du niveau d'activité du patient ou, au contraire, d'une aggravation de l'insuffisance cardiaque.

Par "capteur physiologique" on entendra un capteur censé donner une représentation adéquate des besoins métaboliques du patient à un instant donné, typiquement un capteur de ventilation-minute (capteur VE) selon une technique classique exposée par exemple dans le EP-A-0 151 689.

Par "capteur d'activité" on entendra un capteur permettant de détecter rapidement un changement de l'activité du porteur de l'appareil, typiquement un accéléromètre (capteur G), comme enseigné par exemple dans les EP-A-0 550 293 (Ela Médical) ou EP-A-0 750 920 (Ela Médical), ce dernier document décrivant un stimulateur à double capteur, physiologique et d'activité.

Du point de vue physiologique, on part du principe qu'un patient fait des exercices pour lesquels il y a proportionnalité entre le niveau d'activité, mesuré avec le capteur G, et le niveau physiologique, mesuré avec le capteur VE. Pour chaque effort, on ne retient que la relation entre le niveau maximal moyenné Gmax de l'accélération G et le niveau maximal moyenné VEmax de la ventilation-minute VE. On obtient ainsi pour chaque effort un couple (Gmax, VEmax).

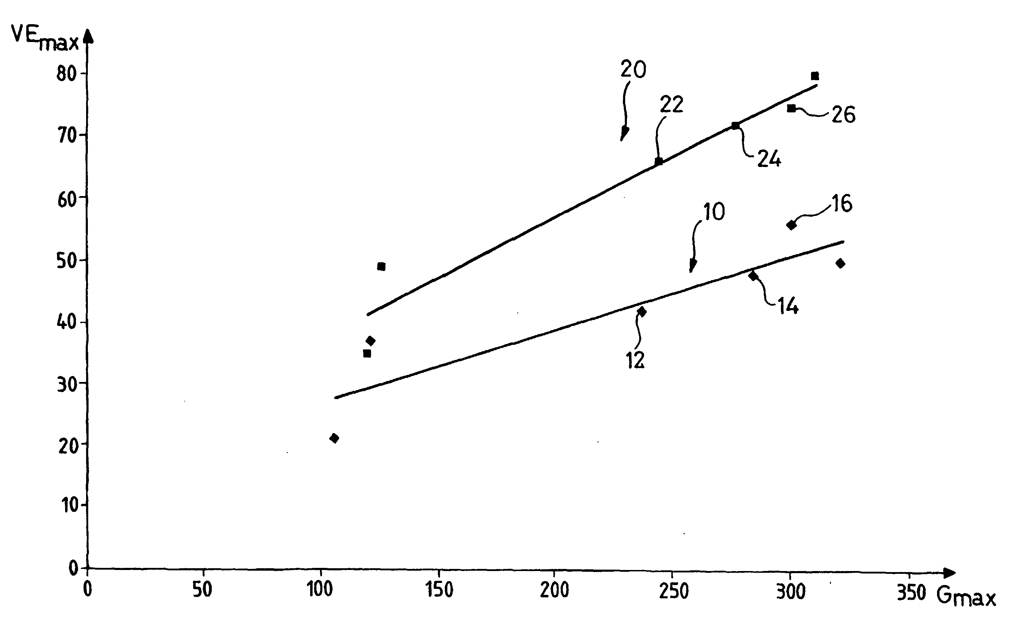

Si l'on représente, comme on l'a fait sur la figure 1, par des points 12, 14, 16 ... ces différents couples relevés au long d'une journée, on obtient du fait de la proportionnalité une courbe qui peut être modélisée par une droite 10 ; si cette relation n'est pas linéaire, la droite ne sera pas optimale, mais la tendance sera respectée (la droite peut être par exemple déterminée par un calcul de régression linéaire).

Or il est connu que, pour un même niveau d'effort (activité G identique), un patient insuffisant cardiaque hyperventilera par rapport à un patient sain. Ainsi, si deux patients comparables, l'un sain l'autre insuffisant cardiaque, atteignent au cours d'un effort le même niveau d'activité (Gmax identique), la valeur VEmax du premier sera inférieure à la VEmax du second (cette affirmation n'étant bien entendu vraie que de façon statistique, pour une population).

Maintenant, si l'on considère le même patient suivi dans le temps, celui-ci est traité à l'aide d'un stimulateur cardiaque, qui compense en partie sa pathologie. Il peut présenter au cours du temps une plus ou moins grande décompensation cardiaque, évoluant en fonction de différents facteurs tels que l'efficacité de la stimulation cardiaque, l'efficacité des médicaments, l'alimentation ou les arythmies auriculaires et/ou ventriculaires. Dans ce cas, d'un jour à l'autre, le patient pourra être comparé à lui-même. Ainsi, pour des phases de décompensation cardiaque croissante, on observera une augmentation de VEmax pour des mêmes niveaux d'effort Gmax. En d'autres termes, le patient aura produit une plus grande ventilation pour le même effort, ce qui signifie que son état s'aggrave.

Si l'on trace les caractéristiques correspondantes d'un jour à l'autre sur le diagramme de la figure 1, on obtient par exemple la caractéristique 10 un jour où l'insuffisance cardiaque est modérée, et une courbe 20 (déterminée à partir des points 22, 24, 26 ... de la même manière que la courbe 10 à partir des points 12, 14, 16 ...) un jour où son insuffisance cardiaque est décompensée.

On constate que dans ce dernier cas la courbe est déplacée vers le haut, c'est-à-dire vers des valeurs de VE plus importantes pour les mêmes niveaux de G.

Le stimulateur peut ainsi recueillir chaque jour une courbe telle que 10 ou 20, et éventuellement déduire de cette courbe un indice caractéristique, tel que le décalage de la caractéristique vers le haut (dans le sens des VEmax croissantes) ou encore, le cas échéant, une variation de la pente de la caractéristique.

Après quelques jours stables, on détermine un indice standard du patient, par rapport auquel une variation brutale de cet indice sera le signe d'une décompensation cardiaque.

Ces informations peuvent être mises en mémoire dans le stimulateur et transmises au praticien par télémétrie au prochain examen clinique du patient, en vue de procéder à une éventuelle reprogrammation du stimulateur.

On peut par ailleurs prévoir, avantageusement, que le stimulateur adapte de lui-même son fonctionnement à la variation du niveau de décompensation.

Dans le cas d'un stimulateur multisite, le stimulateur peut modifier la configuration des sites de stimulation, comme cela est proposé par exemple dans le EP-A-0 925 806 précitée.

## Revendications

1. Un dispositif médical implantable actif asservi, notamment un stimulateur cardiaque, défibrillateur et/ou cardioverteur, comportant :
- au moins un capteur d'effort, mesurant un paramètre (VE) à prépondérance physiologique et délivrant un signal fonction de l'effort développé par un patient porteur du dispositif,
- au moins un capteur d'activité, mesurant un paramètre (G) à prépondérance physique, et
- des moyens d'analyse de tendance, aptes à :
a) mesurer périodiquement par les deux capteurs des couples de valeurs (12, 14, 16 ; 22, 24, 26) correspondant à un niveau d'effort donné développé par le patient ; et
b) établir une caractéristique (10, 20) fonction des couples de valeurs ainsi mesurés ;
**caractérisé en ce que** les moyens d'analyse de tendance sont également aptes à:
c) évaluer le degré de décompensation cardiaque du porteur de l'appareil d'après l'évolution au cours du temps de ladite caractéristique, en recherchant un éventuel accroissement des signaux délivrés par le capteur d'effort par rapport aux signaux délivrés par le capteur d'activité.

2. Le dispositif de la revendication 1, dans lequel les moyens d'analyse de tendance sont également aptes à :
d) modifier la programmation du dispositif lorsque les résultats de l'étape c) indiquent un accroissement au-delà d'un seuil donné des signaux délivrés par le capteur d'effort par rapport aux signaux délivrés par le capteur d'activité.

3. Le dispositif de la revendication 2, dans lequel des électrodes sont placées en une pluralité de sites myocardiques distincts de stimulation et dans lequel la commande de la stimulation et/ou la configuration des sites sont modifiées lorsque les résultats de l'étape c) indiquent un accroissement au-delà d'un seuil donné des signaux délivrés par le capteur d'effort par rapport aux signaux délivrés par le capteur d'activité.

4. Le dispositif de la revendication 1, dans lequel les moyens d'analyse de tendance évaluent à l'étape c) le décalage de la caractéristique dans le sens des efforts croissants.

5. Le dispositif de la revendication 1, dans lequel les moyens d'analyse de tendance évaluent à l'étape c) l'accroissement de la pente effort/activité de la caractéristique.

6. Le dispositif de la revendication 1, dans lequel l'évaluation de l'étape c) est opérée régulièrement, typiquement quotidiennement.

## Patentansprüche

1. Geregelte aktive implantierbare medizinische Vorrichtung, insbesondere Herzschrittmacher, Defibrillator und/oder Kardioverter, umfassend:
- mindestens einen Anstrengungssensor, welcher einen überwiegend physiologischen Parameter (VE) misst und ein Signal liefert, welches abhängig von der Anstrengung eines die Vorrichtung tragenden Patienten ist,
- mindestens einen Sensor der Aktivität, welcher einen überwiegend physischen Parameter (G) misst, und
- Mittel zur Trendanalyse, geeignet, um:
a) durch zwei Sensoren periodisch Wertpaare (12, 14, 16; 22, 24, 26) zu messen, welche einem gegebenen Anstrengungsgrad des Patienten entsprechen; und
b) eine Kennlinie (10, 20) zu erstellen, welche abhängig von den so gemessenen Wertpaaren ist;
**dadurch gekennzeichnet, dass** die Mittel zur Trendanalyse gleichfalls fähig sind:
c) den Grad der kardialen Dekompensation des Trägers des Apparates zu berechnen nach der Entwicklung der genannten Kennlinie im Verlauf der Zeit, indem ein eventueller Zuwachs von durch den Anstrengungssensor gelieferten Signalen im Verhältnis zu den durch den Aktivitätssensor gelieferten Signalen gesucht wird.

2. Vorrichtung gemäß Anspruch 1, in welcher die Mittel zur Trendanalyse gleichfalls geeignet sind:
d) die Programmierung der Vorrichtung zu ändern, wenn die Ergebnisse des Schritts c) einen Zuwachs über eine gegebene Schwelle der durch den Anstrengungssensor gelieferten Signale im Verhältnis zu den durch den Aktivitätssensor gelieferten Signalen anzeigen.

3. Vorrichtung gemäß Anspruch 2, in welcher die Elektroden an einer Vielzahl von verschiedenen myokardischen Stimulationsstellen platziert sind und in welcher die Steuerung der Stimulation und/oder die Konfiguration der Stellen modifiziert werden, wenn die Ergebnisse des Schritts c) einen Zuwachs über eine gegebene Schwelle der durch den Anstrengungssensor gelieferten Signale im Verhältnis zu den durch den Aktivitätssensor gelieferten Signalen anzeigen.

4. Vorrichtung gemäß Anspruch 1, in welcher die Mittel zur Trendanalyse im Schritt c) die Verschiebung der Kennlinie in Richtung der wachsenden Anstrengungen berechnen.

5. Vorrichtung gemäß Anspruch 1, in welcher die Mittel zur Trendanalyse in Schritt c) den Zuwachs der Anstrengungs-/Aktivitätssteigung der Kennlinie berechnen.

6. Vorrichtung gemäß Anspruch 1, in welcher die Berechnung von Schritt c) regelmäßig ausgeführt wird, typischerweise täglich.

## Claims

1. A rate responsive active implantable medical device, in particular a cardiac pacemaker, defibrillator and/or cardioverter, comprising:
- at least one effort sensor, measuring a predominantly physiological parameter (VE) and issuing a signal which is a function of the effort developed by a patient bearing the device,
- at least one activity sensor, measuring a predominantly physical parameter (G) of patient, and
- trend analysis means, adapted to:
a) periodically measure by means of the two sensors couples of values (12, 14, 16 ; 22, 24, 26), corresponding to a given level of effort and activity developed by the patient;
b) determine a characteristic (10, 20) which is a function of the couples of values thus measured;
**characterised in that** the trend analysis means are further adapted to:
c) estimate the cardiac decompensation degree of the bearer of the device from the evolution during time of said characteristic, by searching for a possible increase of the signals issued by the effort sensor with respect to the signals issued by the activity sensor.

2. The device of claim 1, wherein the trend analysis means are further adapted to:
d) modify the programming of the device when the results of step c) indicate an increase beyond a given threshold of the signals issued by the effort sensor with respect to the signals issued by the activity sensor.

3. The device of claim 2, wherein electrodes are placed in a plurality of distinct stimulation myocardial sites, and wherein the control of the stimulation and/or the arrangement of the sites are modified when the results of step c) indicate an increase beyond a given threshold of the signals issued by the effort sensor with respect to the signals issued by the activity sensor.

4. The device of claim 1, wherein the trend analysis means estimate at step c) the shift of the characteristic in the direction of increasing efforts.

5. The device of claim 1, wherein the trend analysis means estimate at step c) the increase in the effort/activity slope of the characteristic.

6. The device of claim 1, wherein the estimation of step c) is performed on a regular basis, typically on a daily basis.
